# FASCICULE DE BREVET EUROPEEN

(11) **EP 2 387 389 B1**
(45) Date de publication et mention de la délivrance du brevet: **08.06.2016**
(21) Numéro de dépôt: 10706700.1
(22) Date de dépôt: 12.01.2010
(51) Int. Cl.: A61K 9/00, A61K 9/08, A61K 31/05, A61K 31/167, A61K 31/5377, A61K 31/553, A61K 47/10, A61P 1/06, A61P 25/04

(54) **FORMULATION POUR L'ADMINISTRATION PAR VOIE TRANS-MUQUEUSE BUCCALE DE MOLECULES ANTALGIQUES ET/OU ANTI-SPASMODIQUES**
FORMULIERUNG FÜR DIE ORALE TRANSMUKOSALE VERABREICHUNG VON ANALGETISCHEN UND/ODER ANTISPASTISCHEN MOLEKÜLEN
COMPOSITION FOR ORAL TRANSMUCOSAL ADMINISTRATION OF ANTALGIC OR ANTISPASMODIC AGENTS

(30) Priorité: 13.01.2009 FR 0950145
(43) Date de publication de la demande: 23.11.2011
(73) Titulaire: Perovitch, Philippe, 33680 Le Temple (FR); Maury, Marc, 33160 Saint Medard en Jalles (FR)
(72) Inventeur: Perovitch, Philippe, 33680 Le Temple (FR); Maury, Marc, 33160 Saint Medard en Jalles (FR)
(74) Mandataire: CAPRI
(86) Numéro de dépôt international: PCT/FR2010/050039
(87) Numéro de publication internationale: WO 2010/081984

(56) Documents cités:
- WO-A-2008/035020
- WO-A-2008/079295
- FR-A- 2 910 317
- FR-A- 2 918 874
- FR-A1- 2 912 654
- US-B1- 6 440 453
- TAKAHASHI KOICHI ET AL: "Novel approach to improve permeation of ondansetron across shed snake skin as a model membrane" JOURNAL OF PHARMACY AND PHARMACOLOGY, ROYAL PHARMACEUTICAL SOCIETY OF GREAT BRITAIN, GB, vol. 53, no. 6, 1 juin 2001 (2001-06-01), pages 789-794, XP008109189 ISSN: 0022-3573
- KRISHNAIAH Y S R ET AL: "Penetration-enhancing effect of ethanolic solution of menthol on transdermal permeation of ondansetron hydrochloride accross rat epidermis" DRUG DELIVERY, ACADEMIC PRESS, ORLANDO, FL, US, vol. 15, no. 4, 1 janvier 2008 (2008-01-01), pages 227-234, XP008109160 ISSN: 1071-7544

## Description

La présente invention concerne une formulation pour l'administration systémique instantanée par voie trans-muqueuse buccale d'au moins un principe actif ayant une action antalgique et/ou anti-spasmodique.

L'invention se rapporte également à un procédé de préparation de cette formulation et à son utilisation pour le traitement et la prévention des crises spastiques et douloureuses.

Certaines pathologies organiques liées à des processus inflammatoires, se manifestent par des épisodes de crises spastiques violentes, imprévisibles et douloureuses, souvent localisées au niveau du système hépato-entéro-colique (colites spasmodiques et cholécystites) ou du système génito-urinaire (coliques néphrétiques et cystites). Il s'agit de spasmes réactionnels des organes qui provoquent des états d'impotence fonctionnelle exigeant parfois un traitement d'urgence, l'absence de thérapeutique pouvant conduire à un état syncopal.

Parmi les traitements utilisés pour atténuer ou lever les spasmes, deux molécules sont particulièrement connues : le Phloroglucinol commercialisé sous le nom de Spasfon® et le Tiémonium commercialisé sous le nom de Viscéralgine®. Ces deux actifs, qui dominent le secteur des antispasmodiques, sont des ammoniums quaternaires, molécules lipophiles de faibles poids moléculaires.

Le Phloroglucinol est un spasmolytique musculotrope, anstispasmodique à action plus particulièrement marquée sur l'uretère et le sphincter d'Oddi. Il agit directement sur la fibre lisse et se comporte comme un antagoniste du calcium au niveau de la membrane musculaire en élevant le 3'5'AMP Cyclique par inhibition de l'enzyme phosphodiestérase.

Le Tiémonium Méthylsulfate est un antispasmodique des manifestations douloureuses aiguës digestives (colites, cholécystite), gynécologiques (dysménorrhées) ou urinaires (coliques néphrétiques). Il possède une double action pharmacologique : une action de type papavérinique musculotrope agissant directement sur les fibres musculaires lisses en se comportant comme le Phloroglucinol, en antagoniste du calcium au niveau de membrane cellulaire, et une action parasympatholytique modérée au niveau des ganglions du système sympathique mais ne devenant ganglioplégique qu'à de très fortes doses.

Outre ces antispasmodiques, dans le cadre d'un traitement de crise spastique, il est également possible d'administrer au patient en souffrance un antalgique, permettant de réduire sa douleur.

Parmi les antalgiques susceptibles d'être administrés en cas de crise spastique, le Néfopam est particulièrement adapté. Il s'agit d'un antalgique pur non opioïde d'action centrale, lipophile ou amphiphile, utilisé dans le traitement des douleurs modérées à intenses en particulier post-opératoires. Il peut potentiellement être utilisé pour le traitement d'urgence des pathologies douloureuses dites « spastiques », telles que les colites spasmodiques, les colites néphrétiques ou des douleurs post-opératoires ou post-traumatiques viscérales. Il présente l'avantage de n'être pas dépresseur respiratoire et de ne pas induire de réduction du transit intestinal.

Si toutes ces molécules utilisées pour traiter des troubles spastiques, aussi bien les molécules antispasmodiques que les antalgiques, sont reconnues comme présentant une activité avérée, leur administration pour traiter une crise spastique présente toutefois de nombreuses difficultés.

Les antalgiques type Néfopam sont principalement administrés sous forme intraveineuse ou intramusculaire.

Les anti-spasmodiques majeurs quant à eux sont généralement administrés par voie orale sous forme de comprimés ou de Lyocs. Ils peuvent également être administrés en suppositoires et sous forme injectable intraveineuse ou intramusculaire.

Le mode d'administration le plus rapide et le plus efficace, quelle que soit la molécule, est la voie intra-veineuse ou intramusculaire. Toutefois ce mode d'administration nécessite un support médical de prescription, l'utilisation de matériels spécifiques et du personnel dédié. Il est d'un coût élevé et sa mise en oeuvre est lourde pour le patient, inadaptée à une automédication.

Dans le cadre d'un traitement de crises spastiques, il est donc préférable d'éviter les formes injectables et d'administrer les molécules à visée antispasmodique ou antalgiques par voie orale.

La forme orale majoritairement connue est l'administration par voie entérale à l'aide de comprimés ou Lyocs, mais ce mode d'administration n'est pas non plus adapté ni aux antispasmodiques ni aux antalgiques.

En effet, tout d'abord les crises spastiques violentes sont souvent associées à des troubles digestifs. Les patients visés peuvent être extrêmement sensibles à toute prise orale de médicaments et les rejeter instantanément.

Outre cette difficulté suivant l'état du sujet et sa capacité d'absorber une forme pharmaceutique sans la rejeter, dans les meilleures conditions d'absorption, il faut compter un délai d'au moins une quarantaine de minutes avant le début d'un effet pharmacodynamique induisant une réduction des spasmes et douleurs, délai démesuré par rapport à l'attente d'un patient en souffrance.

Lorsqu'elles sont introduites dans le tube digestif et l'estomac, les molécules antispasmodiques ou antalgiques de nature lipophile subissent l'effet dit de « premier passage digestif », altérations et déperditions liées au milieu stomacal ou aux variations des physiologies intestinales. Elles sont ensuite soumises à un effet dit de « premier passage hépatique » qui provoque leur métabolisation et/ou leur dégradation plus ou moins intense, avec constitution de nombreux métabolites, pour la plupart inactifs ou toxiques provoquant des effets secondaires.

La dose de principes actifs véritablement biodisponible, résorbée par le tractus digestif est donc extrêmement faible.

Pour le Néfopam elle est quasi nulle, car ses trois principaux métabolites sont tous dépourvus d'activité pharmacologique.

Pour les antispasmodiques, seule une part résiduelle est effectivement distribuée aux membranes musculaires lisses et inhibe l'enzyme phosphodiestérase : en moyenne au mieux 40 à 50% pour le Phloroglucinol et entre 10 et 15% pour le Tiémonium. Ceci est vrai aussi bien pour un comprimé enrobé que pour une forme Lyoc. En effet la forme Lyoc, bien qu'indiquée en usage sublingual, ne permet pas de dissoudre parfaitement les molécules antispasmodiques, toujours amphiphiles ou hydrophobes, dans l'atmosphère buccale, lesdites molécules étant quand même dégluties et métabolisées dans le tractus digestif et le foie.

Il apparaît donc plusieurs problèmes majeurs.

Le premier problème est qu'il faut parvenir à faire absorber un produit à un sujet déjà fragilisé susceptible de réflexes nauséeux. La prise médicamenteuse ne doit pas être rejetée une fois avalée et le principe actif doit être suffisamment absorbé malgré les éventuels troubles digestifs du patient.

Une deuxième difficulté réside dans l'administration d'une dose suffisante de médicament au patient, tenant compte du statut pondéral du sujet, de la dilution et de la dispersion de ce principe actif dans l'organisme, pour que la seule part significativement active atteigne les systèmes hépato-entéro-colique ou génito-urinaire pour les antispasmodiques ou bien le système nerveux central pour les antalgiques.

Une autre problématique est le temps de latence dû à la métabolisation et à la diffusion dans l'organisme avant que la molécule agisse et que le patient en ressente les bienfaits.

L'administration d'antispasmodiques ou d'antalgiques par voie digestive n'est donc pas appropriée. Les biodisponibilités et délais d'action de ces molécules sont en décalage surprenant avec l'urgence de traitement pour laquelle elles sont sollicitées.

Le traitement des pathologies douloureuses organiques spastiques est donc actuellement difficile et problématique et les moyens thérapeutiques disponibles sont inadaptés. Il subsiste un besoin pour une formulation galénique simple de fabrication, d'action rapide, aisément disponible et maniable en toute autonomie par le patient lui-même, qui ne soit ni une forme conventionnelle, trop lente et aléatoire, ni une forme injectable, exclusive du corps médical et infirmier, mais une forme apte à lever le spasme dans le délai le plus bref avec la meilleure économie de dosage. Les documents FR-2 910 317, WO 2008/035020, WO 2008/079295, US-6 440 453 font partie de l'état de la technique.

C'est ce à quoi répond la présente invention en proposant une formulation ou forme galénique spécifique, sous forme de solution très spécifique, permettant de garantir l'administration instantanée par voie trans-muqueuse buccale d'au moins un principe actif antalgique destiné au traitement d'une crise spastique, à dose efficace équivalente à celle d'une dose intraveineuse ou intramusculaire.

L'invention a pout objet une formulation telle que décrite dans la revendication 1.

Des variantes avantageuses sont décrites dans les revendications dépendantes. L'invention propose également un procédé de préparation ainsi que l'utilisation de cette formulation pour le traitement de pathologies spastiques.

Au sens de la présente invention, on entend par crise spastique ou pathologie spastique toute hyperactivité des fibres musculaires lisses organiques, quelle que soit son origine physiopathologique, provoquant des syndromes douloureux invalidants justifiant un geste thérapeutique.

Avantageusement, par rapport aux formulations existantes, la formulation selon l'invention est très simple de fabrication et d'utilisation et permet le passage trans-muqueux buccal instantané et complet d'une préparation thérapeutique à base de molécules antalgiques et/ou antispasmodiques, en limitant toute dilution salivaire et déglutition des molécules qui sont délivrées quasi-instantanément au système vasculaire pour une distribution de la totalité du dosage au niveau des récepteurs spécifiques de leur activité pharmacologique organique. La dose de principe actif administré est en outre plus faible que celle qu'il est nécessaire d'introduire dans les formulations orales existantes.

La formulation est simple d'utilisation, peu onéreuse, facilement disponible et peu invasive. Elle permet d'administrer une quantité immédiatement biodisponible d'antalgiques et/ou d'antispasmodiques, de façon à pouvoir traiter très rapidement et efficacement des crises spastiques. Elle permet également de façon avantageuse, d'associer dans une même formulation un antispasmodique à action périphérique et un antalgique à action centrale.

La formulation selon l'invention est à la fois utile en urgence, pour lever rapidement un spasme à la seule dose efficace, sans avoir à utiliser la voie intraveineuse ou intramusculaire, mais aussi en automédication car elle permet de fournir des doses adaptées.

D'autres caractéristiques et avantages ressortiront de la description qui va suivre de l'invention.

Selon un premier aspect, l'invention a donc pour objet une formulation pour l'administration par voie trans-muqueuse buccale d'au moins un principe actif destiné au traitement d'une crise spastique, comprenant :
- au moins un principe présent sous forme base et/ou sous forme de sel, choisi parmi les antispasmodiques à action périphérique ou le Néfopam,
- une solution hydroalcoolique titrant au moins 35 degrés d'alcool, et
- éventuellement un autre principe actif présent sous forme base et/ou sous forme de sel, choisi parmi les antalgiques à action centrale.

Très préférentiellement la formulation n'est constituée que de ces seuls éléments.

Le ou les principe(s) actif(s) sont présent(s) en état de dissolution stable et complète dans la solution hydroalcoolique, de volume préférentiellement inférieur à 2mL, de façon à permettre une absorption rapide dudit principe actif à travers les muqueuses de la cavité buccale.

L'invention vise donc une formulation comprenant une solution hydroalcoolique comprenant :
- un antispasmodique à action périphérique seul,
- un antispasmodique à action périphérique associé à un antalgique à action centrale, comme le Paracétamol ou le Néfopam,
- du Néfopam seul, ou
- du Néfopam associé à un autre antalgique à action centrale, comme le Paracétamol.

L'appartenance d'une molécule à une catégorie pharmaco-thérapeutique est faite en référence à celle qui lui est attribuée dans son Autorisation de Mise sur la Marché.

En particulier le Phloroglucinol et le Tiémonium figurent dans le Dictionnaire VIDAL à la sous-rubrique « Antispasmodiques », et le Néfopam et le Paracétamol à la sous-rubrique « Antalgiques non-opioïdes ».

Par "voie trans-muqueuse", on entend tout franchissement passif d'une molécule lipophile ou amphiphile présentée en état de dissolution stable à travers les muqueuses linguales, sublinguales, gingivales, palatines, jugales, ou toutes autres muqueuses constitutives de la cavité buccale.

Par "état de dissolution stable et complète", on entend un état de dissolution restituant le principe actif à l'état moléculaire et faiblement ionisé dans son milieu de dissolution, état de dissolution prévenant toute éventualité d'une recristallisation inopportune. Cet état de dissolution stable et complète peut être contrôlé dès la mise en oeuvre de la formulation selon l'invention par évaluation de l'apparence visuelle de la solution obtenue (mesure du degré de limpidité) puis au niveau des résidus de filtration (apparition ou non de cristaux), et enfin à moyen et long terme au cours des tests de suivi de stabilité à températures et degrés d'hygrométrie variables.

Par "solution hydro-alcoolique titrant X degrés d'alcool", on entend une solution présentant un degré d'alcool de X, correspondant au rapport entre le volume d'alcool pur (100°) contenu dans la solution hydroalcoolique et le volume total de cette solution. Le degré d'alcool de la solution hydroalcoolique varie en fonction du degré de l'alcool utilisé pour former la solution et du ratio eau/alcool de la solution. Par exemple pour un alcool initial à 100 degrés et un ratio eau/alcool 50/50, la solution hydroalcoolique titre 50 degrés d'alcool.

Le principe actif antalgique et/ou antispasmodique est présent sous forme base et/ou sous forme de sel, par exemple sous forme de Succinate, de Chlorhydrate, de Sulfate ou de Méthylsulfate.

Le principe actif antalgique est choisi parmi tous les antalgiques non opiacés d'action centrale , à titre d'exemples non limitatifs, on peut citer des principes actifs lipophiles ou amphiphiles comme le Néfopam ou le Paracétamol.

Le principe actif destiné au traitement de crises spasmodiques est préférentiellement choisi parmi tous les antispasmodiques, comme le Phloroglucinol ou le Tiémonium.

De façon préférée, la formulation comprend le Néfopam ou le Paracétamol ou le Phloroglucinol ou le Tiémonium.

Selon un mode de réalisation particulier, la formulation comprend au moins deux principes actifs différents : un antalgique d'action centrale et un antispasmodique d'action exclusivement périphérique, c'est-à-dire viscérale.

L'antalgique est choisi parmi le Néfopam ou le Paracétamol.

L'antispasmodique est choisi parmi le Phloroglucinol ou le Tiémonium.

Cette combinaison couvre simultanément et en une seule administration à faible dosage, les registres, d'habitude distincts, des traitements de la douleur et du spasme.

Ce traitement est à administration unique pour un double champ d'actions thérapeutiques instantané.

Un exemple particulièrement adapté consiste à associer dans la formulation du Néfopam et du Phloroglucinol.

La formulation selon l'invention peut éventuellement comprendre également un agent correcteur de pH. Par agent correcteur de pH on entend tout agent acide ou tout agent basique n'altérant pas les caractères physico-chimiques du ou des principes actifs.

Préférentiellement, l'agent correcteur de pH est choisi parmi les carbonates et bicarbonates de sodium, les phosphates monosodique ou disodique, la triéthanolamine, l'hydroxyde de sodium (NaOH) et la potasse (KOH) mais aussi des agents acides Chlorhydrique, Sulfurique, Phosphorique, Citrique, Malique, Lactique, Succinique et/ou Butyrique.

De façon préférentielle, la formulation selon l'invention se présente sous forme d'une solution hydroalcoolique comprenant entre 30 et 95% d'alcool en volume et une teneur en eau comprise entre 5 et 70%. Encore plus préférentiellement, la formulation selon l'invention se présente sous forme d'une solution hydroalcoolique comprenant entre 40 et 85% d'alcool en volume et une teneur en eau comprise entre 60 et 15%. Cela correspond à des proportions très proches en masse, du fait des masses volumiques de l'eau (environ 1 g/cm³) et de l'éthanol (0,79g/cm³).

La solution hydroalcoolique a un degré d'alcool d'au moins 35°, préférentiellement compris entre 35 et 70°, encore plus préférentiellement entre 35° et 60°, et idéalement autour de 40° à 50°. Ce degré d'alcool est particulièrement adapté aux molécules antalgiques et/ou antispasmodiques et permet leur dissolution constamment stable et complète ainsi que leur absorption totale quasiinstantanée à travers les muqueuses buccales.

Avantageusement, la solution hydroalcoolique est le seul solvant utilisé dans la formulation selon l'invention.

En outre, l'alcool de la solution hydroalcoolique ne joue pas seulement le rôle de diluant, mais également celui de promoteur d'une absorption per-muqueuse accélérée, dont la vitesse croit en fonction de l'élévation du degré d'alcool utilisé. Le degré d'alcool de la formulation ne doit cependant pas dépasser 70° car un degré supérieur serait incompatible avec un produit pharmaceutique à application buccale pour cause de brûlure muqueuse.

Selon un mode de réalisation préféré et tout particulièrement adapté de l'invention, la solution hydroalcoolique est réalisée à base d'eau et d'éthanol.

A titre illustratif, le coefficient de dissolution du Phloroglucinol dans l'éthanol permet d'obtenir une dissolution complète dudit principe actif à hauteur de 20mg de Phloroglucinol pour 0,75 ml d'éthanol à environ 40°.

De même le coefficient de dissolution du Tiémonium méthylsulfate dans l'éthanol permet d'obtenir une dissolution complète dudit principe actif à hauteur de 10mg de Tiémonium pour 0,75 ml d'éthanol à environ 40°. Le coefficient de dissolution du Néfopam dans l'éthanol permet d'obtenir une dissolution complète dudit principe actif à hauteur de 20 mg de Néfopam pour 1 ml d'éthanol à environ 40°. Le coefficient peut être modulé en fonction du degré d'alcool souhaité pour un passage trans-muqueux accéléré et du ratio préférentiel de dissolution eau/éthanol utilisé.

Préférentiellement le pH de la formulation selon l'invention est compris entre 5,0 et 9,0, encore plus préférentiellement entre 5,5 et 7,5. Ces pH sont favorables à une absorption optimale de la solution.

La formulation selon l'invention permet au principe actif de franchir passivement les muqueuses buccales dans un délai inférieur à 6 à 10 secondes après administration. Ce délai d'absorption très rapide permet de prévenir toute stagnation de la solution et du principe actif dans l'atmosphère buccale ainsi que leur mélange inopportun avec de la salive susceptible de les altérer, ce qui introduirait une rupture dans la continuité et la stabilité de la dissolution du ou des principes actifs. Ce court délai permet également de prévenir toute déglutition réflexe de la solution et du principe actif qu'elle contient.

Le passage trans-muqueux buccal du principe actif présenté en état de dissolution selon l'invention du côté de la membrane épithéliale externe, constituée de structures phospho-lipidiques qui absorbent passivement par affinité élective les molécules lipophiles présentées en état de dissolution stable et complète, est basé sur un appel osmotique vers l'autre côté de ladite membrane, auquel participent ensemble la concentration en principe actif dissous et celle de la solution alcoolique considérée. L'appel osmotique est d'autant plus vivace et puissant que le degré d'alcool qui sert de promoteur d'absorption est élevé. Un degré d'alcool adapté pour le Phloroglucinol, le Tiémonium ou le Néfopam est compris entre 35° et 60°, préférentiellement environ 40° à 50°.

Ceci permet d'assurer simultanément l'obtention et le réglage du meilleur coefficient de dissolution et de stabilisation de la molécule ainsi que la promotion de son passage per-muqueux dans un délai de 4 à 10 secondes, pour un volume inférieur ou égal à 1mL.

Un mode de réalisation particulièrement adapté correspond à 0,75ml de solution hydroalcoolique à un degré d'alcool de environ 40° pour 10 mg de Tiémonium méthylsulfate ou pour 20 mg de Phloroglucinol ou pour 10 mg de Néfopam.

Un autre mode de réalisation particulièrement adapté correspond à 1ml de solution hydroalcoolique à un degré d'alcool de environ 40° pour 25 mg de Tiémonium méthylsulfate ou pour 40 mg de Phloroglucinol ou pour 20 mg de Néfopam.

Les muqueuses de la bouche possèdent un réseau très dense de micro-vaisseaux, quasi-spongieux, si bien que les molécules, tant de solvant alcoolique que de principe actif dissous, qui franchissent les pores lipophiles de la membrane épithéliale, sont instantanément capturées par la micro-circulation sanguine et collectées vers les veines sublinguales, puis les veines jugulaires en direction du coeur. Ce phénomène est accentué par la présence de l'alcool qui provoque une vasodilatation et un accroissement du débit micro-vasculaire local des muqueuses.

Du fait de ce débit circulatoire élevé, il n'y a donc jamais d'équilibre de part et d'autre de la membrane épithéliale : la concentration dans la bouche reste toujours plus importante, jusqu'à épuisement du mécanisme par défaut de molécules à absorber.

Ainsi, à la différence notoire de toutes les autres formes dites "sub-linguales", la totalité de l'alcool et du principe actif qui s'y trouve dissous selon l'invention, passe à travers la muqueuse.

L'utilisation de la forme galénique selon l'invention permet d'administrer passivement une dose d'antalgiques et/ou d'antispasmodiques, immédiatement absorbée dès que déposée au contact de la muqueuse, pour être distribuée dans l'instant par voie vasculaire, sans aucun délai pour son action pharmacologique et sans subir les effets préalables destructeurs des passages digestif et hépatique.

La forme galénique selon l'invention permet donc une immédiateté d'absorption tissulaire complète des molécules antalgiques et/ou antispasmodiques, puis leur distribution dans la circulation centrale de l'organisme, générant une réponse pharmacologique rapide de type "flash".

Par exemple, avec une forme galénique selon l'invention, réalisée à partir d'une composition de 40 mg de Phloroglucinol solubilisé dans 1ml d'une solution d'éthanol à 40°, on peut administrer quasi-instantanément et passivement une dose très significative de Phloroglucinol. Cette dose de 40 mg est supérieure à la fraction maximale théoriquement disponible d'une dose normalement administrée par voie orale (62,25 mg) qui est située entre 40 et 50% (31,12 mg) au mieux de la dose usuellement administrée par voie orale.

De même avec une forme galénique selon l'invention réalisée à partir de 10 mg de Tiémonium solubilisé dans 0,75 ml d'une solution d'éthanol à 40°, on peut administrer quasi-instantanément et passivement une dose supérieure à la fraction maximale théoriquement disponible d'une dose normalement administrée par voie orale (50 mg) qui est située entre 10 et 15% (environ 5 mg) au mieux de la dose usuellement administrée par voie orale.

Selon un autre exemple, avec une forme galénique selon l'invention réalisée à partir de 20 mg de Néfopam solubilisé dans 1ml d'une solution d'éthanol à 40°, on peut administrer quasi-instantanément et passivement une dose identique à la fraction normalement administrée par injection intraveineuse (20 mg de Chlorhydrate de Népofam pour 2 ml de solution).

Avec la formulation selon l'invention, la biodisponibilité de la dose administrée par voie per-muqueuse locale est complète.

La solution hydroalcoolique selon l'invention, titrant au moins 35 degrés d'alcool, présente également l'avantage de solubiliser les molécules antalgiques et antispasmodiques bien qu'elles soient lipophiles, ce qui autorise leur absorption per-muqueuse spontanée et de protéger la formulation pharmaceutique vis-à-vis d'une contamination microbiologique sans devoir introduire d'agent(s) de conservation anti-microbien(s).

Ainsi, la solution hydroalcoolique selon l'invention possède une quadruple compétence :
- elle joue le rôle de solvant du principe actif antalgique et/ou antispasmodique destiné à traiter une crise spastique, molécules lipophiles ou amphiphiles et de faible poids moléculaire,
- elle active le passage per-muqueux de ce principe actif dissous ainsi présenté à l'état moléculaire au niveau de la membrane lipophile,
- le degré d'alcool accroît doublement la vitesse d'absorption muqueuse, à la fois par effet osmotique et en suscitant une vasodilatation micro-vasculaire réflexe, qui accélère le débit micro-circulatoire local, et
- elle est son propre agent de stabilité ce qui évite l'utilisation d'additifs conventionnels.

Avantageusement, la présente invention offre une grande simplicité de réalisation et une très bonne stabilité galénique : la solution eau/alcool extrêmement simplifiée garantit la solubilisation du principe actif et permet de faire abstraction des excipients habituellement utilisés pour les préparations pharmaceutiques conventionnelles, y compris les conservateurs.

Elle permet donc à la fois de réduire les coûts de fabrication et de diminuer les risques d'intolérance et les possibles interactions entre principe actif et excipients.

Selon un autre avantage, les délais d'action pharmacodynamique de la forme galénique selon l'invention sont très courts, comparés aux lenteurs d'absorption des médicaments à base d'antispasmodiques existants qui demandent un délai d'attente d'au moins 40 minutes entre la prise du médicament et le début de l'action pharmacologique antispasmodique, anti-douleur.

La délivrance pharmacologique quasi-instantanée peut permettre à un patient de s'administrer lui-même un produit pour un effet équivalent à l'efficacité d'une injection intraveineuse en flash dans la circulation, sans les inconvénients liés à ce type d'administration. Elle permet également de traiter les cas d'urgence en milieu hospitalier sans les exigences de préparation, pose et surveillance d'un cathétérisme veineux et donc sans risque de contamination nosocomiale.

Il s'agit d'une voie d'administration bien meilleure en termes de simplicité et de disponibilité d'administration non traumatique mais aussi de coût unitaire et thérapeutique, comparée aux modes d'administration des molécules existantes destinées à traiter les crises spastiques. Le gain en termes de rapport dose/effet est au moins de 40 à 50%. Avec la formulation selon l'invention on utilise au moins 40 à 50% de dose en moins pour un effet thérapeutique obtenu sans délai. Les molécules antalgiques et/ou antispasmodiques administrées ne rencontrant pas d'obstacle significatif pour leur distribution instantanée par voie artérielle vers les récepteurs cibles des fibres lisses ou du système nerveux central, qu'elles gagnent en quelques secondes, la dose de base administrée est réduite, comparable à la dose biodisponible indispensable pour exercer l'activité pharmacologique requise. La dose de principe actif contenue dans la formulation selon l'invention est donc inférieure aux doses classiquement administrées. Cette dose est bien entendu dépendante de l'effet recherché. Elle est préférentiellement comprise entre 2 mg et 50 mg de principe actif, pour des volumes de solution hydroalcoolique variant de 0,5 ml à 2 ml.

Par ailleurs, la muqueuse buccale disposant d'une surface totale d'absorption extrêmement large, démultipliée par son caractère de tissu villeux plissé, l'administration de la forme galénique selon l'invention est dépourvue d'un quelconque risque de déglutition intempestive ou de fausse route. En effet, elle permet un passage per-muqueux extrêmement rapide qui prévient toute dissolution salivaire ou déglutition du principe actif administré, avec l'avantage de ne pas déstabiliser les muqueuses, avec des éléments ou excipients divers. De plus, la formulation selon l'invention est particulièrement adaptée aux patients souffrant de crises spastiques violentes accompagnées de syndromes nauséeux, car elle évite tout rejet possible par vomissements du médicament administré.

En outre, les effets de l'alcool sont insignifiants. A titre d'exemple, une solution hydroalcoolique d'Ethanol de 0,75 ml à 40° ne saurait produire qu'une alcoolémie circulante inférieure à 0,004 g par litre de sang, selon la formule officielle de référence de Widmark, soit un cent vingt-cinquième de la tolérance légale en France établie à 0,5 g par litre de sang. De plus, le passage initial de la solution alcoolique par voie pulmonaire, doit permettre la quasi-complète élimination de l'éthanol sous forme de vapeur extraite par voie respiratoire et expirée, avant que l'Ethanol puisse se trouver distribué dans l'organisme. Le vecteur alcoolique est donc éliminé en presque totalité à travers le parenchyme respiratoire.

Selon un deuxième aspect, l'invention concerne un procédé de préparation de la formulation.

Un procédé de fabrication de la forme galénique selon l'invention, particulièrement adapté, comprend les étapes suivantes :
- mélanger de l'alcool et de l'eau purifiée et introduire dans ce mélange au moins un principe actif antalgique et/ou antispasmodique,
- agiter la préparation jusqu'à obtention d'une suspension homogène,
- poursuivre l'agitation jusqu'à dissolution complète du principe actif, et
- filtrer.

Selon un mode de réalisation préféré le procédé comprend les étapes suivantes :
- mélanger de l'éthanol et de l'eau purifiée et introduire dans ce mélange du Néfopam ou du Phloroglucinol ou du Tiémonium méthylsulfate,
- agiter la préparation, préférentiellement durant 10 à 60 minutes, jusqu'à obtention d'une suspension homogène et dissolution complète du principe actif, et
- filtrer.

Le procédé peut éventuellement comprendre les étapes suivantes avant filtration :
- introduire progressivement un agent correcteur de pH jusqu'à obtention d'un pH désiré compris entre 5,0 et 8,0,
- poursuivre l'agitation, préférentiellement durant 5 à 30 minutes, jusqu'à dissolution complète du principe actif, et
- ajouter de l'eau si nécessaire pour compléter au volume désiré.

La présente invention peut être utilisée pour l'administration systémique instantanée à doses réduites et utiles d'antalgiques, notamment d'antalgiques non opioïdes comme le Néfopam ou bien d'antispasmodiques, notamment de Pholoroglucinol ou de Tiémonium.

En particulier la formulation selon la présente invention peut être utilisée pour la réalisation d'un médicament pour les traitements des crises spastiques, en particulier des crises violentes comme les colites spasmodiques, les coliques néphrétiques, les cholecystites ou encore des douleurs post-opératoires ou post-traumatiques viscérales pelviennes, gynécologiques ou gynéco-obstétricales par administration trans-muqueuse buccale.

Un tel médicament à administration trans-muqueuse buccale, présente une activité thérapeutique qui lève le spasme et/ou calme la douleur dans un délai très bref et à des doses réduites par rapport aux doses traditionnelles. Un médicament préféré comprend la formulation selon l'invention associant à la fois une molécule antispasmodique à une molécule antalgique centrale non opiacée comme le Néfopam ou le Paracétamol.

La formulation selon l'invention, correspondant à un très faible volume liquidien, est très facile à administrer. Un patient peut aisément la déposer dans sa bouche au contact direct d'une zone muqueuse précise, de surface réduite, buccale, para-gingivale ou sublinguale.

De préférence le patient doit déposer la formulation selon l'invention au niveau d'un territoire muqueux à l'abri des sécrétions salivaires, par exemple la gouttière jugale, délimitée d'une part par la couronne gingivale inférieure et externe et d'autre part par la paroi muqueuse des faces inférieures et internes des joues et lèvre inférieure. Ce canal représente en moyenne un réservoir clos d'environ 18 cm de long et de 1 à 1,5 cm de profondeur, soit une surface d'absorption muqueuse de 35 à 55 cm².

Selon un dernier aspect, la formulation selon l'invention nécessite un conditionnement industriel spécifique, afin de permettre son utilisation sécurisée, simple et ergonomique et de prévenir la dégradation du principe actif au contact de l'air.

Un mode de réalisation particulier consiste à utiliser un conditionnement, préférentiellement de petite taille, plastique ou métalloplastique souple ou en verre, opaque, rempli sous atmosphère inerte tel que de l'azote, pour la protection de la stabilité de la composition et l'imperméabilité à l'oxygène et aux rayonnements. Ces conditionnements garantissent la dissolution et la stabilité dans le temps des principes actifs dissous en solution hydroalcoolique selon l'invention.

Préférentiellement ces conditionnements comportent une canule permettant le dépôt précis de la solution selon l'invention au contact d'une zone muqueuse adéquate.

Pour le confort d'utilisation par le patient, pour un transport aisé, on peut préférentiellement recourir à des emballages sous forme d'étuis étanches spécifiques. Encore plus préférentiellement, la forme galénique selon l'invention est conditionnée dans des emballages unidoses de 0,5 à 2 ml, susceptibles de fournir une dose adéquate de principe actif.

De façon avantageuse, ce conditionnement est facile à transporter et permet une utilisation aisée de la forme galénique à tout moment de la journée.

On peut citer plusieurs exemples de formulation selon l'invention, particulièrement adaptés pour produire une efficacité anti-spasmodique/anti-douleur :

**Formulation 1 : Tiémonium méthylsulfate 25 mg pour 1,00 ml à environ 40° d'Éthanol**

| | |
|---|---|
| - Tiémonium méthylsulfate (principe actif) : | 25,0 mg |
| - alcool éthylique 95° (diluant et promoteur d'absorption) : | 0,40 ml |
| - Eau purifiée (diluant) : | qsp 1,00 ml |

Ce premier exemple de formulation peut être obtenu par la mise en oeuvre du procédé décrit en suivant pour un lot de 1000 doses, soit 1l.

Dans une cuve en acier inoxydable introduire 0,40 l d'éthanol 95% V/V et 0,6 l d'eau purifiée.

Introduire dans la solution hydroalcoolique 25 g de Tiémonium méthylsulfate.

À l'aide d'un agitateur à hélice, agiter la préparation durant 20 à 40 minutes jusqu'à obtention d'une suspension homogène.

Poursuivre l'agitation jusqu'à dissolution complète du Tiémonium.

Filtrer la préparation sur un filtre en polypropylène ou équivalent de 5 microns de porosité et répartir la préparation dans des flacons monodoses de 1,00 ml.

**Formulation 2 : Tiémonium méthylsulfate 10 mg pour 0,75 ml à environ 40° d'Éthanol**

| | |
|---|---|
| - Tiémonium méthylsulfate (principe actif) : | 10,0 mg |
| - alcool éthylique 95° (diluant et promoteur d'absorption) : | 0,30 ml |
| - Eau purifiée (diluant) : | qsp 0,75 ml |

Ce deuxième exemple de formulation peut être obtenu par la mise en oeuvre du procédé décrit en suivant pour un lot de 1000 doses, soit 0,75 l.

Dans une cuve en acier inoxydable introduire 0,30 l d'éthanol 95% V/V et 0,45 l d'eau purifiée.

Introduire dans la solution hydroalcoolique 10 g de Tiémonium méthylsulfate.

A l'aide d'un agitateur à hélice, agiter la préparation durant 20 à 40 minutes jusqu'à obtention d'une suspension homogène.

Poursuivre l'agitation jusqu'à dissolution complète du Tiémonium.

Filtrer la préparation sur un filtre en polypropylène ou équivalent de 5 microns de porosité et répartir la préparation dans des flacons monodoses de 0,75 ml.

**Formulation 3 : Phloroglucinol 20 mg pour 0,75 ml à environ 40° d'Éthanol**

| | |
|---|---|
| - Phloroglucinol (principe actif) : | 20,0 mg |
| - alcool éthylique 95° (diluant et promoteur d'absorption) : | 0,30 ml |
| - Eau purifiée (diluant) : | qsp 0,75 ml |

Cet exemple de formulation peut être obtenu par la mise en oeuvre du procédé décrit en suivant pour un lot de 1000 doses, soit 0,75 l.

Dans une cuve en acier inoxydable introduire 0,30 l d'éthanol 95% V/V et 0,45 l d'eau purifiée.

Introduire dans la solution hydroalcoolique 20 g de Phloroglucinol.

A l'aide d'un agitateur à hélice, agiter la préparation durant 20 à 40 minutes jusqu'à obtention d'une suspension homogène et dissolution complète du Phloroglucinol.

Filtrer la préparation sur un filtre en polypropylène ou équivalent de 5 microns de porosité et répartir la préparation dans des flacons monodoses de 0,75 ml.

**Formulation 4 : Phloroglucinol 40 mg pour 1,00 ml à environ 40° d'Éthanol**

| | |
|---|---|
| - Phloroglucinol (principe actif) : | 40,0 mg |
| - alcool éthylique 95° (diluant et promoteur d'absorption) : | 0,40 ml |
| - Eau purifiée (diluant) : | qsp 1,00 ml |

Cet exemple de formulation peut être obtenu par la mise en oeuvre du procédé décrit en suivant pour un lot de 1000 doses, soit 1,0 l.

Dans une cuve en acier inoxydable introduire 0,40 l d'éthanol 95% V/V et 0,60 l d'eau purifiée.

Introduire dans la solution hydroalcoolique 40 g de Phloroglucinol.

A l'aide d'un agitateur à hélice, agiter la préparation durant 20 à 40 minutes jusqu'à obtention d'une suspension homogène.

Poursuivre l'agitation jusqu'à dissolution complète.

Filtrer la préparation sur un filtre en polypropylène ou équivalent de 5 microns de porosité et répartir la préparation dans des flacons monodoses de 1,0 ml.

**Formulation 5 : Néfopam 20 mg pour 1,00 ml à 40° d'Éthanol**

| | |
|---|---|
| - Néfopam (principe actif) : | 20,0 mg |
| - alcool éthylique 95° (diluant et promoteur d'absorption) : | 0,40 ml |
| - Eau purifiée (diluant) : | qsp 1,00 ml |

Cet exemple de formulation peut être obtenu par la mise en oeuvre du procédé décrit en suivant pour un lot de 1000 doses, soit 1,0 l.

Dans une cuve en acier inoxydable introduire 0,40 l d'éthanol 95% V/V et 0,60 l d'eau purifiée.

Introduire dans la solution hydroalcoolique 20 g de Néfopam.

A l'aide d'un agitateur à hélice, agiter la préparation durant 20 à 40 minutes jusqu'à obtention d'une suspension homogène.

Poursuivre l'agitation jusqu'à dissolution complète.

Filtrer la préparation sur un filtre en polypropylène ou équivalent de 5 microns de porosité et répartir la préparation dans des flacons monodoses de 1,0 ml.

**Formulation 6 : Néfopam 10 mg pour 0,75 ml à 40° d'Éthanol**

| | |
|---|---|
| - Néfopam (principe actif) : | 10,0 mg |
| - alcool éthylique 95° (diluant et promoteur d'absorption) : | 0,30 ml |
| - Eau purifiée (diluant) : | qsp 0,75 ml |

Cet exemple de formulation peut être obtenu par la mise en oeuvre du procédé décrit en suivant pour un lot de 1000 doses, soit 0,75 l.

Dans une cuve en acier inoxydable introduire 0,30 l d'éthanol 95% V/V et 0,45 l d'eau purifiée.

Introduire dans la solution hydroalcoolique 10 g de Néfopam.

A l'aide d'un agitateur à hélice, agiter la préparation durant 20 à 40 minutes jusqu'à obtention d'une suspension homogène et dissolution complète du Phloroglucinol.

Filtrer la préparation sur un filtre en polypropylène ou équivalent de 5 microns de porosité et répartir la préparation dans des flacons monodoses de 0,75 ml.

**Formulation 7 : Néfopam 10 mg - Phloroglucinol 20 mg pour 1,0 ml à 40° d'Éthanol**

| | |
|---|---|
| - Néfopam (principe actif) : | 10,0 mg |
| - Phloroglucinol (principe actif) | 20,0 mg |
| - alcool éthylique 95° (diluant et promoteur d'absorption) : | 0,40 ml |
| - Eau purifiée (diluant) : | qsp 1,00 ml |

Cet exemple de formulation peut être obtenu par la mise en oeuvre du procédé décrit en suivant pour un lot de 1000 doses, soit 1,0 l.

Dans une cuve en acier inoxydable introduire 0,40 l d'éthanol 95% V/V et 0,60 l d'eau purifiée.

Introduire dans la solution hydroalcoolique 10 g de Néfopam et 20 g de Phloroglucinol.

A l'aide d'un agitateur à hélice, agiter la préparation durant 20 à 40 minutes jusqu'à obtention d'une suspension homogène et dissolution complète du Phloroglucinol et du Néfopam.

Filtrer la préparation sur un filtre en polypropylène ou équivalent de 5 microns de porosité et répartir la préparation dans des flacons monodoses de 1,0 ml.

**Formulation 8 : Tiémonium 10 mg - Paracétamol 125 mg pour 1,0 ml à 45° d'alcool**

| | |
|---|---|
| - Tiémonium méthylsulfate (principe actif) : | 10,0 mg |
| - Paracétamol (principe actif) | 125,0 mg |
| - alcool éthylique 95° (diluant et promoteur d'absorption) : | 0,45 ml |
| - Eau purifiée (diluant) : | qsp 1,00 ml |

Cet exemple de formulation peut être obtenu par la mise en oeuvre du procédé décrit en suivant pour un lot de 1000 doses, soit 1,0 l.

Dans une cuve en acier inoxydable introduire 0,45 l d'éthanol 95% V/V et 0,55 l d'eau purifiée.

Introduire dans la solution hydroalcoolique 10 g de Tiémonium méthylsulfate et 125 g de Paracétamol.

A l'aide d'un agitateur à hélice, agiter la préparation durant 20 à 40 minutes jusqu'à obtention d'une solution homogène.

Filtrer la préparation sur un filtre en polypropylène ou équivalent de 5 microns de porosité et répartir la préparation dans des flacons monodoses de 1,0 ml.

**Formulation 9 : Phloroglucinol 40 mg - Paracétamol 125 mg pour 1,0 ml à 45° d'alcool**

| | |
|---|---|
| - Phloroglucinol (principe actif) : | 40,0 mg |
| - Paracétamol (principe actif) | 125,0 mg |
| - alcool éthylique 95° (diluant et promoteur d'absorption) : | 0,45 ml |
| - Eau purifiée (diluant) : | qsp 1,00 ml |

Cet exemple de formulation peut être obtenu par la mise en oeuvre du procédé décrit en suivant pour un lot de 1000 doses, soit 1,0 l.

Dans une cuve en acier inoxydable introduire 0,45 l d'éthanol 95% V/V et 0,55 l d'eau purifiée.

Introduire dans la solution hydroalcoolique 40 g de Phloroglucinol et 125 g de Paracétamol.

A l'aide d'un agitateur à hélice, agiter la préparation durant 20 à 40 minutes jusqu'à obtention d'une solution homogène.

Filtrer la préparation sur un filtre en polypropylène ou équivalent de 5 microns de porosité et répartir la préparation dans des flacons monodoses de 1,0 ml.

Bien entendu, l'invention n'est évidemment pas limitée aux exemples représentés et décrits ci-dessus, mais couvre au contraire toutes les variantes.

## Revendications

1. Formulation pour l'administration par voie trans-muqueuse buccale d'au moins un principe actif destiné au traitement d'une crise spastique, comprenant :
- au moins un principe actif présent sous forme base et/ou sous forme de sel, choisi parmi les antispasmodiques à action périphérique, tel que le Tiémonium ou le Phloroglucinol, ou le Néfopam, et
- une solution hydroalcoolique comprenant entre 40% et 85% en volume d'alcool et entre 15% et 60% en volume d'eau,
le ou les principe(s) actif(s) étant présent(s) en état de dissolution stable et complète dans la solution hydroalcoolique.

2. Formulation selon la revendication 1, comprenant en outre un autre principe actif présent sous forme base et/ou sous forme de sel, choisi parmi les antalgiques à action centrale, tel que le Paracétamol ou le Néfopam.

3. Formulation selon l'une des précédentes revendications, dans laquelle la solution hydroalcoolique titre entre 35 et 70 degrés d'alcool.

4. Formulation selon l'une des précédentes revendications, dans laquelle l'alcool de la solution hydroalcoolique est l'éthanol.

5. Formulation selon l'une des précédentes revendications, comprenant également un agent correcteur de pH.

6. Formulation selon la revendication 5, dans laquelle l'agent correcteur de pH est choisi parmi les carbonates et bicarbonates de sodium, les phosphates monosodique ou disodique, la triéthanolamine, l'hydroxyde de sodium, la potasse et/ou parmi des agents acides Chlorhydrique, Sulfurique, Succinique, Butyrique, Phosphorique, Citrique, Malique et/ou Lactique.

7. Formulation selon l'une des précédentes revendications, présentant un pH compris entre 5,0 et 9,0.

8. Procédé de préparation d'une formulation selon l'une des revendications 1 à 7, **caractérisé en ce qu'**il comprend les étapes suivantes :
- mélanger de l'alcool et de l'eau purifiée et introduire dans ce mélange au moins un principe actif choisi parmi les antispasmodiques à action périphérique, tel que le Tiémonium ou le Phloroglucinol, ou le Néfopam,
- agiter la préparation jusqu'à obtention d'une suspension homogène,
- poursuivre l'agitation jusqu'à dissolution complète du principe actif, et
- filtrer.

9. Procédé de préparation selon la revendication 8, comprenant les étapes suivantes :
- mélanger de l'éthanol et de l'eau purifiée et introduire dans ce mélange d'une part du Néfopam ou du Paracétamol et d'autre part du Phloroglucinol ou du Tiémonium,
- agiter la préparation, préférentiellement durant 10 à 60 minutes, jusqu'à obtention d'une suspension homogène et dissolution complète du principe actif, et
- filtrer.

10. Utilisation de la formulation selon l'une des revendications 1 à 7, pour la réalisation d'un médicament destiné au traitement par administration trans-muqueuse buccale des crises spastiques douloureuses.

11. Utilisation selon la revendication 10, pour la réalisation d'un médicament destiné au traitement des colites spasmodiques, des colites néphrétiques, des douleurs post-opératoires ou post-traumatiques viscérales, pelviennes, gynécologiques ou gynéco-obstétricales par administration trans-muqueuse buccale.

## Patentansprüche

1. Formulierung zur bukkalen transmukosalen Verabreichung von zumindest einem Wirkstoff zur Behandlung eines krampfartigen Anfalls, umfassend:
- zumindest einen Wirkstoff vorliegend in Form einer Base und/oder in Form von Salz, ausgewählt aus peripher wirkenden Antispasmodika, wie etwa Tiemonium oder Phloroglucin oder Nefopam, und
- einer hydroalkoholischen Lösung, aufweisend zwischen 40 und 85 Volumenprozent Alkohol und zwischen 15 und 60 Volumenprozent Wasser,
wobei der Wirkstoff oder die Wirkstoffe in Form einer stabilen und vollständigen Lösung in der hydroalkoholischen Lösung vorliegen.

2. Formulierung nach Anspruch 1, ferner umfassend einen weiteren Wirkstoff vorliegend in Form einer Base und/oder in Form von Salz, ausgewählt aus den zentral wirkenden Analgetika, wie etwa Paracetamol oder Nefopam.

3. Formulierung nach einem der vorstehenden Ansprüche, wobei die hydroalkoholische Lösung einen Alkoholgehalt von 35 bis 70 enthält.

4. Formulierung nach einem der vorstehenden Ansprüche, wobei der Alkohol der hydroalkoholischen Lösung Ethanol ist.

5. Formulierung nach einem der vorstehenden Ansprüche, ebenfalls umfassend ein pH-Korrekturmittel.

6. Formulierung nach Anspruch 5, wobei das pH-Korrekturmittel ausgewählt ist aus den Carbonaten und Bicarbonaten von Natrium, Monatriumphosphat oder Dinatriumphosphat, Triethanolamin, Natriumhydroxid, Kalium und/oder aus Chlorwasserstoffsäuren, Schwefelsäuren, Succinylsäuren, Buttersäure, Phosphorsäure, Zitronensäure, Apfelsäure und/oder Milchsäure.

7. Formulierung nach einem der vorstehenden Ansprüche, aufweisend einen pH-Wert zwischen 5,0 und 9,0.

8. Verfahren zur Zubereitung einer Formulierung nach einem der Ansprüche 1 bis 7, **dadurch gekennzeichnet, dass** es die folgenden Schritte umfasst:
- Mischen des Alkohols und des gereinigten Wassers und Zugeben in diese Mischung mindestens einen Wirkstoff ausgewählt aus den peripher wirkenden Antispasmodika wie etwa Tiemonium oder Phloroglucin oder Nefopam,
- Hin- und Herbewegen der Zubereitung, bis eine homogene Suspension erhalten wird,
- Fortsetzen der Bewegung bis zur vollständigen Auflösung des Wirkstoffs und
- Filtern.

9. Verfahren zur Zubereitung nach Anspruch 8, umfassend die folgenden Schritte:
- Mischen des Ethanols und des gereinigten Wassers und Zugeben in diese Mischung von Nefopam oder Paracetamol einerseits und von Phloroglucin oder Tiemonium andererseits,
- Hin- und Herbewegen der Zubereitung, bevorzugt für die Dauer von 10 bis 60 Minuten, bis zum Erhalt einer homogenen Suspension und vollständigen Auflösung des Wirkstoffs, und
- Filtern.

10. Verwendung der Formulierung nach einem der Ansprüche 1 bis 7 für die Herstellung eines Medikaments zur transmukosalen, bukkalen Verabreichung für die Behandlung von schmerzhaften Krämpfen.

11. Verwendung nach Anspruch 10 zur Herstellung eines Medikaments für die Behandlung von krampfartiger Kolitis, nephrotischer Kolitis, postoperativen Schmerzen oder posttraumatischen viszeralen Schmerzen, Schmerzen im Becken, gynäkologischen oder Geburtshilfliche-Schmerzen, durch bukkale, transmukosale Verabreichung.

## Claims

1. A formulation for oral transmucosal administration of at least one active principle for treating a spastic crisis, including:
• at least one active principle present in base form and/or in salt form, chosen from peripheral action antispasmodics, such as Tiemonium or Phloroglucinol, or Nefopam, et
• an aqueous alcohol solution comprising between 40% and 85% by volume of alcohol, and between 15% and 60% by volume of water,
the active principle(s) being present in a state of stable and complete dissolution in the aqueous alcohol solution.

2. A formulation according to claim 1, further comprising another active principle present in base form and/or in salt form, chosen from central action analgesics, such as Paracetamol or Nefopam.

3. A formulation according to any preceding claim, wherein the aqueous alcohol solution titrates 35° to 70° alcohol.

4. A formulation according to any preceding claim, wherein the alcohol of the aqueous alcohol solution is ethanol.

5. A formulation according to any preceding claim, also including a pH corrector agent.

6. A formulation according to claim 5, wherein the pH corrector agent is chosen from carbonates and bicarbonates of sodium, monosodium or disodium phosphates, triethanolamine, sodium hydroxide, and potassium hydroxide, and/or Hydrochloric, Sulfuric, Succinic, Butyric, Phosphoric, Citric, Malic, and/or Lactic acid agents.

7. A formulation according to any preceding claim, having a pH from 5.0 to 9.0.

8. A method of preparing a formulation according to any one of claims 1 to 7, **characterized in that** it comprises the following steps:
• mixing alcohol and purified water and introducing into this mixture at least one active principle chosen from peripheral action antispasmodics, such as Tiemonium or Phloroglucinol, or Nefopam,
• stirring the preparation until a homogeneous suspension is obtained;
• further stirring to complete dissolution of the active principle; and
• filtering.

9. A preparation method according to claim 8, comprising the following steps:
• mixing ethanol and purified water and introducing into this mixture on one hand Nefopam or Paracetamol and on the other hand Phloroglucinol or Tiemonium;
• stirring the preparation, preferably for 10 min to 60 min, until a homogeneous suspension and complete dissolution of the active principle are obtained; and
• filtering.

10. Use of the formulation according to any one of claims 1 to 7 for the production of a medication intended for the treatment by oral transmucosal administration of painful spastic crises.

11. Use according to claim 10, for the production of a medication intended for treating spasmodic colitis, nephritic colitis, post-operative or post-trauma, pelvic, gynecological, or gyneco-obstetric visceral pain by oral transmucosal administration.
